# EUROPÄISCHE PATENTSCHRIFT

(11) **EP 0 342 402 B1**
(45) Veröffentlichungstag und Bekanntmachung des Hinweises auf die Patenterteilung: **13.07.1994**
(21) Anmeldenummer: 89107546.7
(22) Anmeldetag: 26.04.1989
(51) Int. Cl.: A61B 17/28

(54) **Medizinisches Instrument**
Medical instrument
Instrument médical

(30) Priorität: 17.05.1988 DE 3816798
(43) Veröffentlichungstag der Anmeldung: 23.11.1989
(73) Patentinhaber: WISAP GESELLSCHAFT FÜR WISSENSCHAFTLICHEN APPARATEBAU MBH, D-82054 Sauerlach (DE)
(72) Erfinder: Semm, Kurt, o.Prof. Dr.med. Dr.med.vet.h.c., D-2300 Kiel 1 (DE)
(74) Vertreter: Weber, Otto Ernst, Dipl.-Phys.

(56) Entgegenhaltungen:
- EP-A- 0 119 405
- DE-A- 3 630 210
- US-A- 3 334 630
- US-A- 4 043 323

## Beschreibung

Die Erfindung betrifft ein medizinisches Instrument gemäß Oberbegriff des Anspruchs 1.

Ein derartiges gattungsgemäßes Instrument ist z. B. aus der US-PS 4,043,323 bekannt. Bei diesem bekannten Instrument sind distalseitig Zangenglieder und proximalseitig zwei, zueinander verschiebbare Griffglieder angebracht. Eine Schließbewegung der Zangenglieder wird durch ein Verschieben des vorderen proximalen Griffgliedes in Richtung des zum Instrumentengrundkörper stationär gehaltenen, rückwärtigen Griffgliedes erreicht.

Die Kraftausübung bei diesem bekannten Instrument auf die distalseitigen Zangenglieder ist dadurch begrenzt, daß diese lediglich durch den Daumen und zwei Finger aufgebracht werden kann. Desweiteren besitzt das Instrument eine geringe Stabilität, da die Schwenkachse der Zangenglieder, welche die gesamten axialen Zugkräfte aufnehmen muß, an einer flexiblen Röhre befestigt ist. Insgesamt besteht das Instrument aus relativ vielen Einzelteilen und ist kompliziert aufgebaut, was entsprechend hohe Herstellungskosten zur Folge hat. Ein weiterer Nachteil dieses Instrumentes besteht darin, daß ein Teil der Betätigungseinrichtung der Zangenglieder, insbesondere der Hebelmechanismus, ohne eine Schutzumhüllung am Instrument angeordnet ist. Hierdurch kann es beim Einsatz des Instrumentes im Körper zu Funktionsbeeinträchtigungen kommen, da Gewebe die Betätigungseinrichtung der Zangenglieder blockieren kann.

Bei diesem beschriebenen Instrument kann somit weder eine hohe Kraft auf die distalseitigen Zangenglieder aufgebracht werden, noch kann eine einwandfreie Funktion, trotz eines aufwendigen Instrumentenaufbaus, gewährleistet werden.

Ein weiteres, vergleichbares medizinisches Instrument ist z.B. aus der DE-OS 36 30 210 A1 bekannt. Dieses zuletzt genannte Instrument ist insbesondere im Hinblick auf die Drehbarkeit der proximalen Griffglieder ausgebildet, so daß durch diese Drehbarkeit auch die distalseitigen Glieder, die z. B. als Zangenglieder ausgebildet sein können, bei Bedarf in eine geeignetere Arbeitsposition gedreht werden können. Bei diesem Instrument ist das vordere proximale Griffglied an der Instrumentenhülse starr befestigt, während das hintere proximale Griffglied gegenüber der Hülse verschiebbar gelagert ist. Durch eine Abstandsverkleinerung zwischen den beiden Griffgliedern wird eine Öffnungsbewegung der distalseitigen Zangenglieder erreicht, wobei die anschließende Schließbewegung durch eine vorgespannte Feder ausgeführt wird.

Zwar sind die Griffglieder bei diesem bekannten Instrument als Griffscheiben ausgebildet. Eine ergonomisch optimale Betätigung in Bezug auf eine hohe Kraftausübung ist jedoch nicht möglich, da die axiale Verschiebung bei der Schließbewegung allein durch die vorgegebene Federkraft bewirkt wird.

Ausgehend von diesen Nachteilen im Stand der Technik liegt der Erfindung die **Aufgabe** zugrunde, ein vorgenanntes medizinisches Instrument, das ein Greif-, Klemm- oder Schneidinstrument sein kann, so auszubilden, daß relativ hohe Kräfte auf die distalseitigen Instrumentenglieder übertragbar sind und bei dem jederzeit eine einwandfreie und zuverlässige Funktion sichergestellt ist. Zusätzlich soll ein stabiler und einfacher Instrumentenaufbau erreicht werden, mit dem sowohl der Instrumentendurchmesser als auch der Herstellungsaufwand reduziert wird.

Diese Aufgabe wird erfindungsgemäß durch die Merkmale des kennzeichnenden Teils des Anspruchs 1 gelöst.

Ein wesentlicher Grundgedanke der Erfindung kann darin gesehen werden, von der bisher üblichen Verschiebbarkeit des rückwärtigen proximalen Griffgliedes abzugehen und stattdessen dieses sozusagen stationär zu halten, während über des vordere Griffglied durch eine axiale Schließbewegung zwischen den beiden Griffgliedern eine höhere Kraft auf die distalen Instrumentenglieder aufgebracht werden kann. Die distalen Instrumentenglieder können dabei prinzipiell beliebiger Art sein, z.B. Greif-, Klemm-, Schneid- oder Zangenglieder, so daß die Griffglieder nahezu universell bei medizinischen Instrumenten dieser Art einsetzbar sind.

Um die vorstehende Bewegung der Griffglieder realisieren zu können, ist das rückwärtige Griffglied mit einer Instrumentenhülse starr verbunden. Das vordere Griffglied steht mit einer in der Instrumentenhülse axial zumindest geringfügig verschiebbaren Betätigungsstange mit einer Anlenkung für das oder die distalen Instrumentenglieder in Verbindung, so daß durch eine Axialverschiebung der Betätigungsstange nach vorne eine Öffnungsbewegung und durch axiale Rückwärtsverschiebung die Schließbewegung des Instruments ausgeführt wird. Dies bedeutet, daß der distalseitige Bereich des vorderen Griffgliedes von den Fingern des Operateurs erfaßt werden kann und durch Rückwärtsziehen, also einem Zusammendrücken der beiden Griffglieder, die Schließkraft auf die distalen Instrumentenglieder, z.B. Zangenglieder oder Scherblätter, erhöht werden kann. Hierbei liegt die rückwärtige Seite des rückwärtigen Griffgliedes gegen die Innenhandfläche an.

Bei dem erfindungsgemäßen medizinischen Instrument, dessen Konstruktionsprinzip multifunktionell eingesetzt werden kann und z.B. für Greif-, Klemm- oder Schneidinstrumente geeignet ist, braucht daher die bei Scheren übliche Orientierung der Finger bzw. der menschlichen Hand nicht mehr eingehalten zu werden. Es ist im Gegensatz hierzu die entsprechende Funktion des medizinischen Instrumentes durch Druck oder Zug der Finger in jeder "Dreh"-Lage des Instrumentes, also um 360° funktionsgerecht ausführbar. Die ergonomische Betätigungsweise ermöglicht es daher dem Operateur ohne Änderung der Fingerorientierung, die üblicherweise eine entsprechende Hand- bzw. Unterarm- Orientierung nach sich ziehen würde, mühelos eine Betätigung des medizinischen Instrumentes um 360° ohne jegliche Funktionseinschränkung durchzuführen. Es wird daher für das medizinische Instrument, das z.B. ein Greif-, Klemm- oder Schneidinstrument sein kann, eine Betätigungsmöglichkeit der entsprechenden distalseitigen Instrumentenglieder ermöglicht, unabhängig davon ob deren Bewegung eine Druck- oder Zugbewegung erforderlich macht, ohne daß eine geänderte Hand- bzw. Unterarm-Orientierung erforderlich ist. Die bedienende Person kann daher die für die Hand ergonomisch optimale Position einnehmen, so daß eine nahezu ermüdungslose, unverkrampfte Handhabung des medizinischen Instrumentes möglich ist.

Aus Vereinfachungsgründen wird daher im nachfolgenden das multifunktionelle Instrument als medizinisches Instrument bezeichnet, wobei exemplarisch die distal betätigbaren Instrumentenglieder Zangenglieder genannt werden.

Um sicherzustellen, daß das chirurgische Instrument beim Einführen in den Operationsbereich auf alle Fälle geschlossen ist, kann eine Feder vorgesehen sein, die das vordere Griffglied in der Schließ- bzw. Ruhelage der Zangenglieder nach rückwärts vorspannt. Beim Öffnen der Zangenglieder müßte daher das vordere Griffglied in axialer Richtung nach vorne verschoben werden. Sofern die Federkraft zum Erfassen oder Abschneiden eines Gewebebereichs nicht ausreicht, kann die Erhöhung der Greif-, Klemm-, Schneid- oder Zangenkraft wie vorausgehend beschrieben aufgebracht werden.

In einer einfachen Ausführungsform des Instrumentes ist ein Zangenglied starr mit der Instrumentenhülse verbunden, während nur das zweite Zangenglied direkt oder indirekt über eine Hebelanlenkung betätigbar ist. Sofern als Alternative beide Zangenglieder bewegt werden sollen, ist selbstverständlich die Anlenkung über ein entsprechendes Mittelglied möglich. Die scherenartige Beaufschlagung beider Zangenglieder, die z.B. über einen Parallelogramm-Mechanismus erfolgen kann, ermöglicht eine bessere Kraftbeaufschlagung, da hierbei eine Hebelarmverbesserung vorhanden ist.

Das bewegbare Zangenglied ist zwechmäßigerweise an einer in der Hülse befestigten Schwenkachse gelagert. Die Anlenkund der Betätigungsstange am bewegbaren Griffglied erfolgt zweckmäßigerweise auf der gegenüberliegenden Seite zur Öffnungsbewegung, bezogen auf das Schwenklager.

Anstelle der Betätigungsstange können auch andere Steuerelemente, wie z.B. ein Instrumentenschaft, ein Bautenzug ähnliches eingesetzt werden.

Die kraftschlüssige Kopplung zwischen dem vorderen Griffglied, das zweckmäßigerweise als Griffscheibe ausgebildet ist und der Betätigungsstange erfolft durch einen weitgehend radialen Mitnehmereingriff, der in einer axialen Ausnehmung der Instrumentanhülse verschiebbar ist. Der Mitnehmereingriff kann hierbei direkt durch einen radial in der Innenbohrung der Griffscheibe angeordneten Stift realisiert werden. Im Falle der Verbesserund der Gleitbewegung auf der Instrumentenhülse, wofür das Griffglied auf einer Buchse befestigt ist, kann der Mitnehmer z.B. durch eine radial durch die Buchse und die Betätigungsstange geführte Schraube, insbesondere eine Senk- oder Madenschraube, verwirklicht sein.

Die Gestaltung der Griffglieder als Griffscheiben gestattet die über 360° mögliche Ergreifbarkeit und eine ergonomische Lage der Griffglieder in der Hand. Zur Einstellung der Federkraft ist es vorteilhaft, das Widerlager zur Abstützung der Feder axial auf der Instrumentenhülse verschiebbar festlegen zu können.

Im Hinblick auf die Kraftübertragung und einen kurzen axialen Verschiebeweg steht das bewegbare Zangenglied hebelarmmäßig mit einem daran im stumpfen oder rechten Winkel angreifenden zweiten Hebelarm für die Anlenkung der Betätigungsstange in Verbindung. Zweckmäßigerweise ist hierfür ein einstückiges Zangenglied vorgesehen. Bei der Betätigung nur eines Zangengliedes wird der Abstand zwischen der Drehachse dieses Zangengliedes und dem Anlenkungspunkt des Zangengliedes maximal gehalten. Anlenkungs- und Drehpunkt werden daher geeigneterweise in den Bereich des Maximaldurchmessers der Instrumentenhülse gelegt. Auf diese Weise erreicht man auch hierbei eine günstige Hebelarmwirkung zur Kraftübertragung auf das eine betätigbare Zangenglied.

Die Erfindung wird nachfolgend anhand zweier schematischer Ausführungsbeispiele noch näher erläutert. Das erfindungsgemäße medizinische Instrument, das als Greif-, Klemm-, Schneid- oder Zangeninstrument einsetzbar ist, wird nachfolgend aus Vereinfachungsgründen als Zangeninstrument bezeichnet, wobei dies im Sinne eines Oberbegriffs die verschiedensten Ausführungsformen des Instrumentes umfaßt.

Es zeigen:
- Fig. 1: einen axialen Schnitt durch eine erste Ausführungsform eines erfindunfgsgemäßen medizinischen Instrumentes, z.B. als Zangeninstrument, wobei die Zangenglieder geschlossen sind;
- Fig. 2: bruchstückartig den distalen Bereich des in Fig. 1 gezeigten Instrumentes bei geöffneten Zangengliedern und
- Fig. 3: einen vergleichbaren axialen Schnitt durch ein zweites erfindungsgemäßes Instrument, das eine Federvorspannung aufweist, bei dem die Zangenglieder in Ruhestellung geschlossen sind.

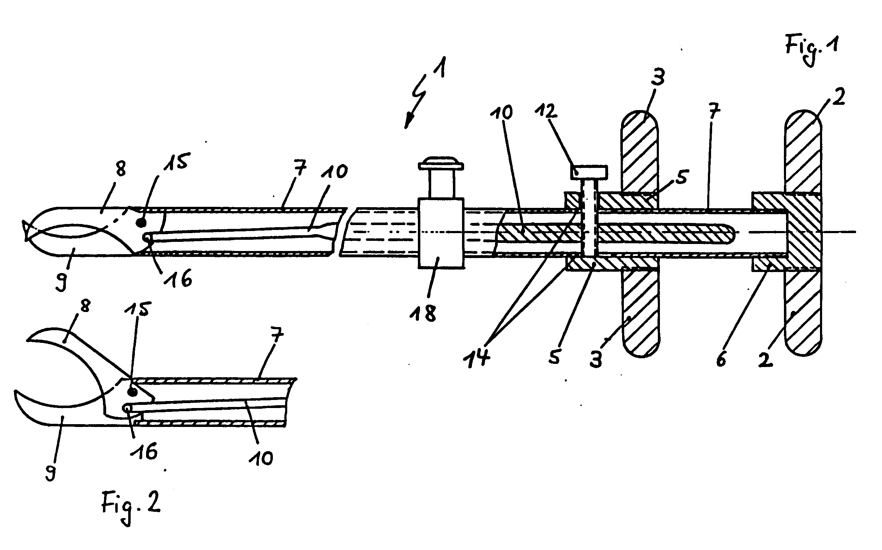

Das in Fig. 1 in einem Axialschnitt dargestellte Zangeninstrument 1 weist im wesentlichen eine rohrförmige Instrumentenhülse 7 auf, in der eine Betätigungsstange 10 mindestens über eine geringe axiale Strecke verschiebbar angeordnet ist. Am rückwärtigen Ende dieser Instrumentenhülse 7 ist eine Blindbuchse 6, z.b. verschraubt, aufgebracht. Auf dieser Buchse 6 ist die rückwärtige Griffscheive 2 starr befestigt, wofür ebenfalls eine Schraubverbindung vorgesehen sein kann.

Am distalen Ende ist ein Zangenglied 9 starr an der Instrumentenhülse, beispielsweise mittels Verschweißen, vorgesehen. Das zweite, im Beispiel obere Zangenglied 8 ist um eine Schwenkachse 15 verschwenkbar. Die Schwenkachse 15 kann mit der Instrumentenhülse 7 vernietet sein.

Während im Beispiel nach Fig. 1 das Schwenklager 15 geringfügig oberhalb der Längsmittelachse vorgesehen ist, greift unterhalb dieses Schwenklagers die Betätigungsstange 10 an einer Anlenkung 16 am Zangenglied 8 an. Die Anlenkung 16 weist üblicherweise ebenfalls eine Achse auf, um die der rückwärtige Teil des Zangengliedes 8 geringfügig bewegbar ist. Im geschlossenen Zustand der Zangenglieder 8, 9, wie wie in Fig. 1 gezeigt sind, fluchten diese in axialer Richtung mit der Außenfläche der Instrumentenhülse 7. Der rückwärtige Teil, also Verschwenkungsteil, des bewegbaren Zangengliedes 8 entspricht etwa dem Innendurchmesser der Instrumentenhülse 7, wobei die proximalseitige Kante abgerundet ausgeführt ist, damit auch bei einer Öffnungsbewegung kein Überstand über den Außendurchmesser der Instrumentenhülse 7 vorliegt.

Die leicht sichelförmig gestalteten Zangenglieder 8 und 9 nach den Fig. 1 und 2 können beispielsweise Teile einer chirurgischen Hakenschere sein.

Zur axialen Verschiebbarkeit der Betätigungsstange 10 ist mit Abstand zum rückwärtigen Griffglied ein ebenfalls auf einer Buchse 5 befestigtes vorderes Griffglied 3 z.B. über Gewinde vorgesehen. Die Kraftübertragung bzw. starre Ankopplung zwischen dem vorderen Griffglied 3 und der Betätigungsstange 10 erfolgt mittels einer etwa radial durch die Buchse und den rückwärtigen Teil der Betätigungsstange 10 geführten Schraube 12. Die Schraube 12 steht zwechmäßigerweise sogar in Eingriff mit der gegenüberliegenden Innenfläche der Buchse 5. Die axiale Verschiebbarkeit gegenüber der stationär gehaltenen Instrumentenhülse 7 wird dadurch ermöglicht, daß auf beiden Seiten der Instrumentenhülse eine axiale Aussparung 14 bzw. entsprechende Schlitze in der Dimension des Schraubendurchmessers vorhanden sind.

Das Zangeninstrument nach Fig. 1 wird an seinen Zangengliedern 8,9 dadurch geöffnet, daß der axiale Abstand zwischen dem vorderen und hinteren Griffglied vergrößert wird. Durch ein Verschieben des Griffgliedes 3 zum distalen Ende auf der Instrumentenhülse 7 wird die Betätigungsstange 10 durch die starre Kopplung nach vorne bewegt. Über die Anlenkung 16 wird dementsprechend eine Hebelbewegung auf das bewegbare Zangenglied 8 ausgeübt, so daß dieses in die in Fig. 2 gezeigte Stellung geöffnet werden kann. Die Betätigungsstange 10, die im rückwärtigen Bereich, insbesondere im Bereich des Mitnehmerstiftes, durchmesserstärker ausgebildet ist, erfährt im vorderen Bereich eine Materialabschwächung mit leichter Auslenkung aus der Längsmittelachse, um im Anlenkungspunkt 16 mit Abstand zur Mittelachse und der Schwenkachse 15 angreifen zu können. In Fig. 2 ist erkennbar,daß der rückwärtige Teil des bewegbaren Zangengliedes 8 so gestaltet ist, daß die Öffnungs- und Schließbewegung im Durchmesserbereich der Instrumentenhülse 7 erfolgen kann.

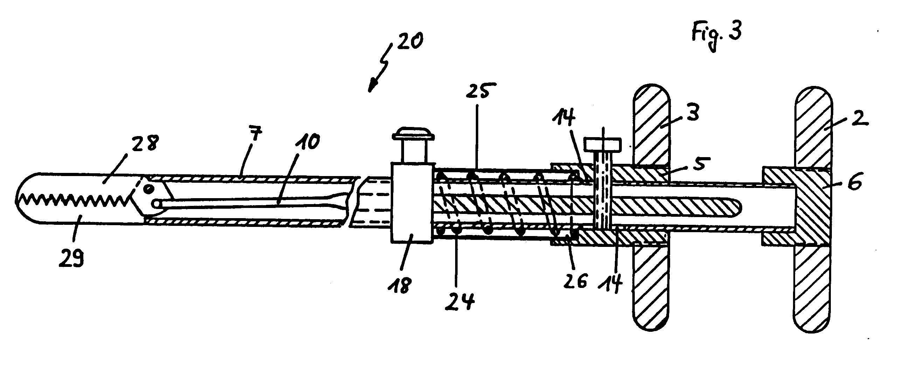

Im zweiten Ausführungsbeispiel des Zangeninstrumentes 20 nach Fig. 3 sind für gleiche Bauelemente und Baugruppen wie nach Fig. 1 gleiche Bezugszeichen verwandt. Der wesentliche Unterschied bei diesem zweiten Ausführungsbeispiel des Zangeninstrumentes 20 besteht darin, daß in der Schließlage der Zangenglieder 28,29 das vordere Griffglied 3 nach rückwärts federvorgespannt ist. Die entsprechende Feder 24 stützt sich dabei gegen ein auf der Instrumentenhülse 7 axial verstellbares, aber arretierbares Widerlager 18 ab. Dieses Widerlager 18 kann als verschiebbarer Ring gestaltet sein. Die Feder 24 ist im Beispiel nach Fig. 3 um die Instrumentenhülse 7 angeordnet, wobei sie von einer Außenhülse 25 umschlossen ist. Diese Außenhülse 25 liegt fest oder beweglich gegen das Widerlager 18 an und erstreckt sich nach rückwärts in eine Ringnut 26 der Buchse 5.

Die im Beispiel nach Fig. 3 gezeigten Zangenglieder 28 und 29 weisen eine sägezahnförmige Kontur auf, wie sie z.B. bei Peritonealscheren erforderlich ist.

Im übrigen entspricht der Aufbau des Beispiels nach Fig. 3 dem nach Fig. 1. In der Schließstellung nach Fig. 3 ist daher das vordere Griffglied durch die Feder 24 nach rückwärts vorgespannt. Zum Öffnen der Zangenglieder 28,29 bedarf es daher einer axialen Verschiebung des Griffgliedes 3 zum distalen Ende der Instrumentenhülse 7. Wesentlich ist aber auch hier, daß für den Fall, daß die Federkraft in der Umsetzung als auf die Zangenglieder 28,29 einwirkende Kraft nicht ausreicht, das vordere Griffglied 3 durch ein Verschieben des Griffgliedes 3 nach rückwärts, was einer Schließbewegung der Hand entspricht, manuell bewegt werden kann, so daß eine erhöhte Zangenkraft zur Durchtrennung z.B. eines Gewebes erreicht wird.
Das medizinische Zangeninstrument besteht im wesentlichen im Hinblick auf die Sterilisierbarkeit aus V2A-Stahl oder ähnlichem Material. Die Griffglieder 2 und 3, die geeigneterweise als Griffscheiben ausgebildet sind, können im ergonmomischen Sinn gestaltet sein, so daß das rüchwärtige Griffglied etwa der Innenhandfläche angeformt ist, während die distale Fläche und der Randbereich des vorderen Griffgliedes zur Erfassung und Betätigung mittels der Finger entsprechende Griffbereiche oder Vertiefungen aufweisen können.

Der konstruktive Bereich dieses Instrumentes ist dabei nicht auf reine Zangeninstrumente, wie Hakenschere, Peritonealschere, Biopsie- und Faßzange, atraumatischen Greifer, Krallengreifer etc. ausgerichtet, sondern betrifft generell die Konstruktion ein- oder beidgliedriger Spreizinstrumente im medizinischen Bereich.

Die Ausstattung des Zangeninstrumentes mit kreisförmigen Griffscheiben als Griffglieder 2 und 3 hat den großen Vorteil, daß bei einer zwangsweise erforderlichen Drehung der distalen Zangenglieder 28,29 aufgrund der medizinischen Gegebenheiten im Operationsbereich, das gesamte Zangeninstrument beispielsweise im Trokar gedreht werden kann. Auch nach einer derartigen operativ erforderlichen Drehund des Instrumentes findet der Operateur gleiche ergonomische Bedingungen aufgrund der scheibenförmig und insbesondere kreisförmigen Griefglieder vor. Gerade im Hinblick auf herkömmliche scherenartige Griffglieder am proximalen Ende von Zangeninstrumenten und der manuellen Erhöhung der Zangenkräfte durch Rückwärtsziehen des vorderen Griffgliedes, zeigt sich der wesentliche Vorteil gegenüber bekannten Zangeninstrumenten.

## Patentansprüche

1. Medizinisches Instrument
mit distalen, z.B. als Zangenglieder gestalteten Instrumentengliedern und proximalseitig zwei axial relativ zueinander bewegbaren Griffgliedern, von denen das rückwärtige proximale Griffglied (2) mit einer Instrumentenhülse verbunden ist, während das andere vordere proximale Griffglied (3) mindestens axial verschiebbar mit einer Betätigungseinrichtung für die Instrumenten- oder Zangenglieder kraftschlüssig gekoppelt ist,
wobei eine axiale Abstandsvergrößerung gegenüber dem rückwärtigen Griffglied (2) die Instrumenten- oder Zangenglieder (8,9; 28,29) öffnet und durch eine axiale Abstandsverkleinerung bei geöffnetem Instrument (1,20) eine kraftbeaufschlagte Schließbewegung der Instrumenten- oder Zangenglieder (8,9; 28,29) herbeiführbar ist,
dadurch **gekennzeichnet**,
(a) daß das Instrument eine im wesentlichen rohrförmige Instrumentenhülse (7) aufweist,
(b) daß das rückwärtige proximale Griffglied (2) starr am proximalen Ende der Instrumentenhülse (7) befestigt ist und diese abschließt,
(c) daß die Zangenglieder (8,9; 28,29) am distalen Ende der Instrumentenhülse (7) angebracht sind,
(d) daß das vordere proximale Griffglied (3) axial verschiebbar auf der Instrumentenhülse (7) gleitet,
(e) daß die Betätigungseinrichtung als Betätigungsstange (10) ausgebildet, die starr mit dem vorderen proximalen Griffglied gekoppelt und in der Instrumentenhülse (7) angeordnet ist, und
(f) daß die Griffglieder (2,3) als Griffscheiben ausgebildet sind und die rückwärtige Griffscheibe (2) bei Betätigung gegen die Innenhandfläche zu liegen kommt.

2. Instrument nach Anspruch 1
dadurch **gekennzeichnet**,
daß ein Zangenglied (9,29) starr am distalseitigen Ende der Instrumentenhülse (7) vorgesehen ist, und das andere bewegbare Zangenglied (8,28) eine Anlenkung (16) mit der Betätigungsstange (10) aufweist.

3. Instrument nach Anspruch 1 bis 2,
dadurch **gekennzeichnet**,
daß das bewegbare Zangenglied (8,28) ein an der Instrumentenhülse (7) befestigtes Schwenklager (15) aufweist und die Anlenkung (16) der Betätigungsstange (10) an einem Hebelarm des bewegbaren Zangengliedes (8; 28) erfolgt.

4. Instrument nach einem der Ansprüche 1 bis 3,
dadurch **gekennzeichnet**,
daß die Betätigungsstange (10) über ein radiales Mitnehmerglied (12), insbesondere einen Stift, mit dem vorderen Griffglied (3) verbunden ist.

5. Instrument nach einem der Ansprüche 1 bis 4,
dadurch **gekennzeichnet**,
daß das Mitnehmerglied (12) in einer Aussparung (14) der Instrumentenhülse (7) axial verschiebbar, insbesondere geführt, ist.

6. Instrument nach einem der Ansprüche 1 bis 5,
dadurch **gekennzeichnet**,
daß das vordere Griffglied (3) in der Ruhelage bzw. Schließlage der Zangenglieder (28,29) nach rückwärts federvorgespannt (24) ist.

7. Instrument nach Anspruch 6,
dadurch **gekennzeichnet**,
daß die Feder (24) an einem Widerlager (18) der Instrumentenhülse (7) abgestützt ist.

8. Instrument nach einem der Ansprüche 1 bis 7,
dadurch **gekennzeichnet**,
daß die am Schwenklager (15) angreifenden Hebelarme des bewegbaren Zangengliedes (8;28) abgewinkelt, insbesondere unter einem Winkel von 60 bis 90°, zueinander vorgesehen sind.

9. Instrument nach einem der Ansprüche 7 bis 8,
dadurch **gekennzeichnet**,
daß die Widerlager (18) zur Einstellung der Federkraft axial verstellbar auf der Instrumentenhülse (7) angeordnet sind.

10. Instrument nach einem der Ansprüche 1 bis 9,
dadurch **gekennzeichnet**,
daß die Schwenklager (15) und die Anlenkung (16) der Betätigungsstange (10) am Hebelarm des bewegbaren Zangengliedes (8) bezogen auf den Durchmesser der Instrumentenhülse (7) einen maximalen Abstand aufweisen.

11. Instrument nach einem der Ansprüche 1 bis 10,
dadurch **gekennzeichnet**,
daß am proximalen Ende der Instrumentenhülse (7) eine Blindbuchse aufgebracht ist, an welcher das Griffglied (2) starr befestigt ist.

12. Instrument nach einem der Ansprüche 1 bis 11,
dadurch **gekennzeichnet**,
daß das vordere proximale Griffglied (3) auf einer Buchse (5) befestigt ist, welche auf der Instrumentenhülse (7) gleitet.

## Claims

1. A medical instrument with distal instrument members, which are e.g. designed as forceps members, and on the proximal side two axially relatively reciprocally movable gripping members, whereof the rear proximal gripping member (2) is connected to an instrument sleeve, whilst the other front proximal gripping member (3) is coupled in non-positive manner so as to be at least axially displaceable with an operating means for the instrument or forceps members, wherein an axial spacing increase with respect to the rear gripping member (2) opens the instrument or forceps members (8, 9; 28, 29) and an axial spacing decrease with the instrument (1, 20) open brings about a force-operated closing movement of the instrument or forceps members (8, 9; 28, 29),
**characterized** in that
(a) the instrument has a substantially tube-like instrument sleeve (7),
(b) the rear proximal gripping member (2) is rigidly attached to the proximal end of the instrument sleeve (7) and terminates the latter,
(c) the forceps members (8, 9; 28, 29) are located at the distal end of the instrument sleeve (7),
(d) the front proximal gripping member (3) axial-displaceably slides on the instrument sleeve (7),
(e) the operating means is constructed as an operating rod (10), which is rigidly coupled with the front proximal gripping member and is arranged in the instrument sleeve (7), and
(f) the gripping members (2, 3) are constructed as gripping disks and the rear gripping disk (2) comes to rest at the palm when operated.

2. An instrument according to claim 1,
**characterized** in that
one forceps member (9, 29) is provided rigidly on the distal end of the instrument sleeve (7) and the other movable forceps member (8, 28) has an articulation point (16) with the operating rod (10).

3. An instrument according to claim 1 or 2,
**characterized** in that
the movable forceps member (8, 28) has a pivot bearing (15) fixed to the instrument sleeve (7) and the articulation point (16) of the operating rod (10) is on a lever arm of the movable forceps member (8; 28).

4. An instrument according to one of the claims 1 to 3,
**characterized** in that
the operating rod (10) is connected by means of a radial driving member (12), in particular a pin, to the front gripping member (3).

5. An instrument according to one of the claims 1 to 4,
**characterized** in that
the driving member (12) is axially displaceable, in particular guided in a recess (14) in the instrument sleeve (7).

6. An instrument according to one of the claims 1 to 5,
**characterized** in that
the front gripping member (3) is rearwardly biased by means of a spring (24) in the inoperative or closed position of the forceps members (28, 29).

7. An instrument according to claim 6,
**characterized** in that
the spring (24) is supported on an abutment (18) of the instrument sleeve (7).

8. An instrument according to one of the claims 1 to 7,
**characterized** in that
the lever arms acting on the pivot bearing (15) of the movable forceps member (8; 28) are provided to form an angle with each other, in particular an angle of roughly 60 to 90°.

9. An instrument according to one of the claims 7 to 8,
**characterized** in that
the abutments (18) are arranged in axially adjustable manner on the instrument sleeve (7) for adjusting the spring tension.

10. An instrument according to one of the claims 1 to 9,
**characterized** in that
the pivot bearing (15) and the articulation point (16) of the operating rod (10) on the lever arm of the movable forceps member (8) have a maximum spacing relative to the instrument sleeve (7) diameter.

11. An instrument according to one of the claims 1 to 10,
**characterized** in that
at the proximal end of the instrument sleeve (7) is mounted a dummy jack, to which the gripping member (2) is rigidly attached.

12. An instrument according to one of the claims 1 to 11,
**characterized** in that
the front proximal gripping member (3) is attached to a bush (5), which slides on the instrument sleeve (7).

## Revendications

1. Instrument médical comportant des organes d'instrumentation à distance, par exemple des organes de pincement et deux organes de manoeuvre, du côté proche, mobiles axialement l'un par rapport à l'autre et dont le plus en arrière (2), est solidairement fixé sur le corps de l'instrument, tandis que l'autre organe de manoeuvre (3) situé le plus en avant peut au moins se déplacer axialement sous l'action d'un dispositif de commande des organes d'instrumentation ou de pincement auquel il est accouplé par liaison mécanique, de sorte que lorsque l'organe (3) s'éloigne de l'organe (2), les organes d'instrumentation ou de pincement (8, 9, ; 28, 29) s'ouvrent pour se refermer lorsque, l'instrument (1, 20) étant ouvert, on provoque cette fermeture en rapprochant les organes (2) et (3),
caractérisé en ce que :
a) le corps de l'instrument est essentiellement constitué par une douille tubulaire (7),
b) l'organe de manoeuvre (2) situé le plus en arrière est fixé rigidement à l'extrémité de la douille (7), qu'il obture,
c) les organes de pincement (8, 9, ; 28, 29) sont montés sur l'extrémité éloignée de la douille (7),
d) l'organe de manoeuvre (3) situé en avant glisse en coulissant axialement sur la douille (7),
e) le dispositif de manoeuvre est constitué d'une tige (10) reliée rigidement à l'organe de manoeuvre (3) situé en avant et logée dans la douille (7)
f) les organes de manoeuvre (2, 3) sont des disques dont celui (2), situé en arrière, est en appui contre l'intérieur de la main, quand on manoeuvre l'instrument.

2. Instrument selon la revendication 1, caractérisé en ce qu'un organe de pincement (9, 29) est monté fixe à l'extrémité éloignée de la douille (7), tandis que l'autre organe de pincement (8, 28), mobile, est relié à la tige de manoeuvre (10) par une articulation (16).

3. Instrument selon les revendications 1 et 2, caractérisé en ce que l'organe de pincement mobile (8, 28 ) est équipé d'un palier d'articulation (15) fixé à la douille (7) tandis que l'articulation (16) de la tige de manoeuvre (10), crée sur l'organe de pincement mobile (8; 28) un bras de levier.

4. Instrument selon une des revendications 1 à 3, caractérisé en ce que la tige de manoeuvre (10) est reliée à l'organe avant (3) de manoeuvre par un entraînement (12 ) disposé radialement, en particulier une broche.

5. Instrument selon une des revendications 1 à 4, caractérisé en ce que l'entraînement (12) peut se déplacer axialement dans une lumière (14) de la douille (7), avec guidage notamment.

6. Instrument selon une des revendications 1 à 5, caractérisé en ce que l'organe avant de manoeuvre (3), lorsqu'il est en position de repos, c'est-à-dire lorsque les organes de pincement (28, 29) sont fermés, est sollicité en direction de l'arrière par un ressort (24).

7. Instrument selon la revendication 6, caractérisé en ce que le ressort (24) s'appuie sur une butée (18) de la douille (7).

8. Instrument selon une des revendications 1 à 7, caractérisé en ce que les bras de levier articulés au palier (15) de l'organe mobile de pincement (8; 28), font en particulier un angle de 60 à 90° l'un par rapport à l'autre.

9. Instrument selon une des revendications 7 à 8, caractérisé en ce que les butées (18) peuvent être déplacées axialement sur la douille (7), afin de régler la force du ressort (24).

10. Instrument selon une des revendications 1 à 9, caractérisé en ce que le palier de basculement (15) et l'articulation (16) de la tige de manoeuvre (10) définissant le bras de levier de l'organe mobile de pincement (8) sont éloignés au maximum, en fonction du diamètre de la douille (7).

11. Instrument selon une des revendications 1 à 10, caractérisé en ce que l'extrémité proche de la douille (7) porte une douille borgne sur laquelle est fixé rigidement l'organe de manoeuvre (2).

12. Instrument selon une des revendications 1 à 11, caractérisé en ce que l'organe avant de manoeuvre (3) est monté sur une douille (5) coulissant sur la douille de l'instrument (7).
